# DEMANDE DE BREVET EUROPEEN

(11) **EP 3 892 202 A1**
(43) Date de publication de la demande: **13.10.2021**
(21) Numéro de dépôt: 21166945.2
(22) Date de dépôt: 06.04.2021
(51) Int. Cl.: A61B 8/12, A61B 8/00

(54) **DISPOSITIF D'IMAGERIE MÉDICALE TEL QUE NOTAMMENT UNE SONDE ETO POUR UNE DÉSINFECTION PAR RAYONNEMENT UV ET PROCÉDÉ DE CONCEPTION**

(30) Priorité: 07.04.2020 FR 2003462
(71) Demandeur: Germitec, 94200 Ivry Sur Seine (FR)
(72) Inventeur: DESHAYS, Clément, 75006 PARIS (FR); NEVEU, Cédric, 92120 MONTROUGE (FR); LÉPINE, Frédéric, 95210 SAINT-GRATIEN (FR)
(74) Mandataire: Lavoix

(57) **Abrégé**

Ce dispositif d'imagerie médicale (1) tel que notamment une sonde ETO, adapté pour être désinfecté au moins à niveau intermédiaire DNI/HLD dans un système de désinfection par rayonnement UV, est caractérisé en ce qu'il comporte au moins une partie (3) réalisée en un matériau transparent aux UV pour réduire les zones d'ombre sur le dispositif.

## Description

La présente invention concerne un dispositif d'imagerie médicale tel que notamment une sonde ETO et un procédé de conception d'un tel dispositif médical.

Plusieurs techniques de désinfection de ce type de dispositifs d'imagerie médicale sont connues dans l'état de la technique.

La plus simple de ces techniques consiste à nettoyer le dispositif à l'aide d'une lingette et de produits de désinfection appropriés.

Par ailleurs il existe plusieurs niveaux de désinfection bien définis par la classification de SPAULDING : en fonction de son usage, un dispositif/instrument correspond à un niveau de criticité, qui correspond lui-même à un niveau de performance microbiologique.

Ainsi en France, un instrument peut être Non Critique (contacte une peau intacte), Semi-Critique (contacte une peau lésée ou une muqueuse), ou Critique (contacte un tissu stérile ou le sang).

Un instrument Non Critique doit être désinfecté avec un produit efficace sur les bactéries uniquement (Désinfection de Niveau Bas DNB), un instrument Semi-Critique avec un produit efficace sur les bactéries, les mycobactéries, les virus, les champignons (Désinfection de Niveau Intermédiaire DNI), et un instrument Critique doit être stérilisé (Stérilisation) ou, si la stérilisation n'est pas possible, au moins désinfecté avec un produit aussi efficace pour les spores (Désinfection de Niveau Haut DNH).

Du point de vue International, il s'agit d'exigences similaires mais exprimées par les termes « Low-Level Disinfection » LLD pour Désinfection de Niveau Bas, « High Level Disinfection » HLD pour Désinfection de Niveau Haut (qui correspond à la DNI Française), et « Sterilization » pour Stérilisation (il n'y a pas de DNH définie hors de France, elle est une spécificité locale).

Ces efficacités contre des familles de microorganismes sont à leur tour encadrées par des normes Françaises, Européennes et Internationales.

Ainsi par exemple, les instruments utilisés dans des situations dites semi-critiques, c'est-à-dire en contact avec une muqueuse ou une peau lésée, doivent notamment en France, subir une Désinfection de Niveau Intermédiaire DNI, et à l'International, une Désinfection de Haut Niveau dite « High Level Disinfection » HLD.

Ces deux niveaux sont identiques, malgré la différence apparente des termes, en ce qu'ils exigent une efficacité totale sur bactéries, champignons, mycobactéries, virus, et une efficacité partielle sur les spores.

Ce niveau DNI/HLD est le minimum visé par la présente invention.

Plusieurs techniques permettent d'atteindre ce niveau de désinfection sur les dispositifs/instruments médicaux d'imagerie médicale.

Une technique classique consiste à utiliser un bain de produit chimique de désinfection dans lequel est trempé le dispositif d'imagerie afin de le désinfecter.

Une autre technique connue consiste à mettre en œuvre un système de désinfection par rayonnement/radiation UV et notamment par UV-C, permettant d'obtenir au moins une désinfection DNI/HLD rapide du dispositif.

Une telle désinfection DNI/HLD par UV revient à faire en sorte que l'ensemble de la surface du dispositif médical à désinfecter, reçoive une dose d'UV suffisante pour atteindre cette désinfection et à contrôler pendant le cycle de désinfection, que la dose prévue a bien été délivrée.

Ceci revient également à dire que l'élément de surface du dispositif qui reçoit le moins de dose d'UV, appelé également point froid, par opposition à point chaud qui est lui bien exposé, doit tout de même recevoir une dose d'UV suffisante pour atteindre la désinfection souhaitée et qu'un système de monitorage en temps réel des cycles de désinfection associé est en mesure de connaitre la dose d'UV délivrée sur ledit point froid.

Mais des essais ont montré que certains dispositifs sont peu adaptés pour permettre une telle désinfection en ce qu'ils comportent de par leur forme, des zones d'ombre, ou points froids, très prononcés, ce qui revient à dire que leur configuration géométrique présente des zones très peu accessibles au rayonnement UV émis dans une chambre de désinfection classique.

Le but de l'invention est donc de résoudre ces problèmes.

A cet effet l'invention a pour objet un dispositif d'imagerie médicale tel que notamment une sonde ETO, adapté pour être désinfecté au moins à niveau intermédiaire DNI/HLD dans un système de désinfection par rayonnement UV, caractérisé en ce qu'il comporte au moins une partie réalisée en un matériau transparent aux UV pour réduire les zones d'ombre sur le dispositif.

Suivant d'autres caractéristiques du dispositif selon l'invention prises seules ou en combinaison :
- ladite partie du dispositif est réalisée en un matériau transparent à au moins 30% de transmission du rayonnement UV ;
- ladite partie du dispositif permet de réduire d'au moins 15% les zones d'ombre du dispositif ;
- ladite partie du dispositif est réalisée en quartz ;
- ladite partie du dispositif est réalisée en polyfluoroéthylène ;
- ladite partie du dispositif comprend au moins un organe de manipulation de ce dispositif ;
- il est adapté pour atteindre la désinfection DNI/HLD en moins de 10 mn dans un système à chambre UV qui délivre une dose cumulée d'au moins 60 mJ/cm² sur au moins une des autres surfaces du dispositif durant le cycle de désinfection ;
- ladite partie du dispositif permet de réduire les écarts d'exposition de ses différentes portions de plusieurs ordres de grandeur.

Selon un autre aspect l'invention a également pour objet un procédé de conception d'un dispositif médical tel que notamment une sonde ETO comme décrit précédemment, caractérisé en ce qu'il consiste à caractériser les zones d'ombre du dispositif, et à concevoir et valider la conception de celui-ci à l'aide d'un outil de simulation optique assistée par ordinateur.

L'invention sera mieux comprise à la lecture de la description qui va suivre, donnée uniquement à titre d'exemple et faite en se référant aux dessins annexés, sur lesquels :
[Fig. 1] la figure 1 représente une vue de côté d'un exemple de réalisation d'un dispositif d'imagerie médicale dans un système de désinfection par rayonnement UV ; et

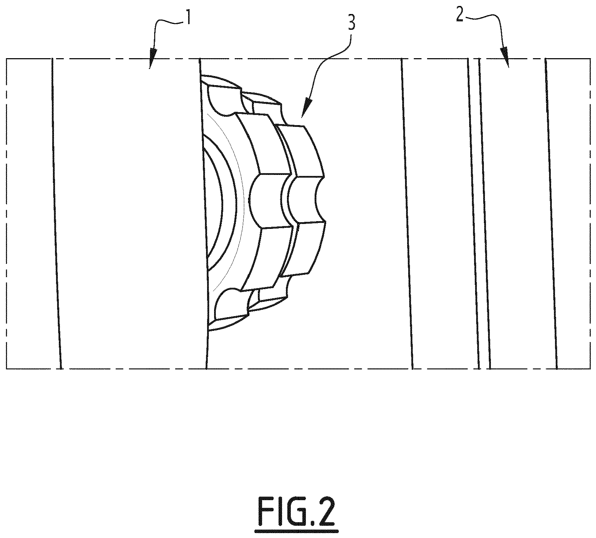

[Fig. 2] la figure 2 représente une vue de détail de ce dispositif.

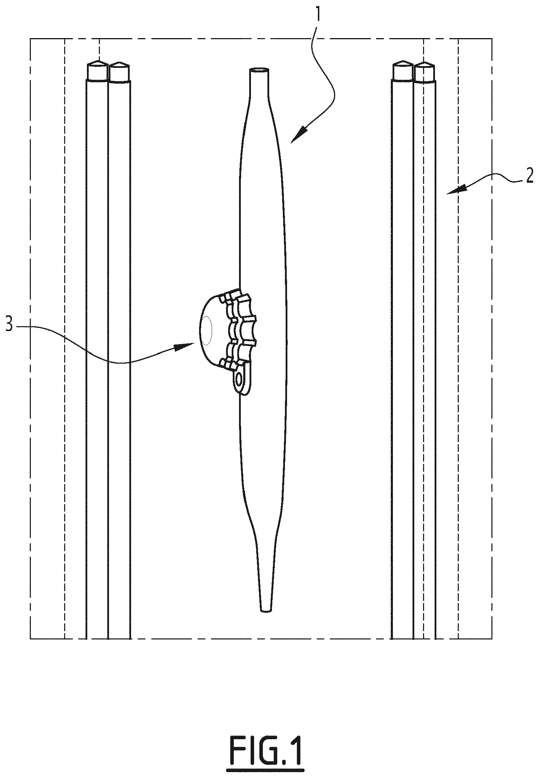

On a en effet illustré sur ces figures et en particulier sur la figure 1, un dispositif d'imagerie médicale qui se présente par exemple sous la forme d'une sonde ETO désignée par la référence générale 1 sur ces figures.

Ce dispositif est adapté pour être désinfecté à un niveau DNI/HLD dans un système de désinfection par rayonnement UV, et notamment par UV-C, schématisé sur cette figure et désigné par la référence générale 2.

Comme cela est illustré, ce dispositif d'imagerie médicale comporte par exemple des organes de manipulation, désignés par la référence générale 3, qui sont susceptibles de former des zones d'ombre sur le dispositif, ces zones d'ombre étant également appelées points froids, ne recevant pas une dose d'UV suffisante pour atteindre la Désinfection DNI/HLD .

Ces zones d'ombre du dispositif peuvent par exemple être caractérisées par un outil de simulation optique assistée par ordinateur ou par toute autre technique.

Cet outil peut également servir à concevoir et à valider la conception d'un tel dispositif pour vérifier que le dispositif conçu est bien à même d'être désinfecté comme requis et permettre au système de monitorage en temps réel des cycles de désinfection, de calculer la dose reçue par et au travers de la partie transparente aux UV.

Ces différentes opérations entrent alors dans le cadre d'un procédé global de conception et de validation du dispositif.

Pour résoudre ces problèmes de zones d'ombre, le dispositif d'imagerie médicale selon l'invention comporte au moins une partie réalisée en un matériau transparent aux UV pour réduire ces zones d'ombre sur le dispositif.

Dans l'exemple illustré sur ces figures, la ou les parties réalisées en matériau transparent aux UV du dispositif, sont des organes de manipulation de celui-ci, c'est-à-dire par exemple les éléments désignés par la référence générale 3.

Ces parties du dispositif sont alors réalisées en un matériau transparent à au moins 30% de transmission du rayonnement UV.

Le matériau utilisé peut par exemple être du quartz ou encore du polyfluoroéthylène.

Bien entendu d'autres matériaux et d'autres parties du dispositif réalisées dans ce type de matériau encore peuvent être envisagés.

On conçoit alors que l'utilisation de ce matériau pour réaliser au moins certaines pièces ou parties du dispositif, pouvant provoquer des zones d'ombre sur le dispositif, permet d'exposer correctement le maximum de la surface du dispositif médical afin d'obtenir sur toute cette surface, la désinfection DNI/HLD souhaitée.

Ainsi ladite partie du dispositif réalisée dans ce matériau transparent au UV, permet de réduire d'au moins 15% les points froids ou zones d'ombre par rapport à une configuration identique qui aurait été faite en un matériau bloquant les UV.

Elle permet de réduire dans ce sens les écarts d'exposition entre les points chauds et les points froids du dispositif de plusieurs ordres de grandeur (moins de 5x, moins de 4x, moins de 3x, moins de 2x).

Ceci permet alors d'utiliser un système de désinfection par UV classique permettant d'obtenir une désinfection DNI/HLD en un temps limité d'exposition de quelques minutes comme moins de 10 mn par exemple, dans un système à chambre UV qui délivre une dose cumulée d'au moins 60 mJ/cm² sur au moins une des autres surfaces du dispositif durant le cycle de désinfection.

Ainsi on peut concevoir que, dans le dispositif selon l'invention, le point froid recevra au moins un cinquième de la dose reçue par le point chaud lors d'une désinfection par un appareil classique, le point froid étant au moins deux fois plus chaud que ce qu'il aurait été si un matériau autre qu'un matériau transparent aux UV avait été utilisé pour réaliser la ou les parties concernées du dispositif.

Ceci peut alors être validé comme décrit précédemment en mettant en œuvre l'outil de simulation optique mentionné précédemment.

Bien entendu d'autres modes de réalisation encore peuvent être envisagés.

## Revendications

1. Dispositif d'imagerie médicale (1) tel que notamment une sonde ETO, adapté pour être désinfecté au moins à niveau intermédiaire DNI/HLD dans un système de désinfection par rayonnement UV (2), **caractérisé en ce qu'**il comporte au moins une partie (3) réalisée en un matériau transparent aux UV pour réduire les zones d'ombre sur le dispositif.

2. Dispositif médical tel que notamment une sonde ETO selon la revendication 1, **caractérisé en ce que** ladite partie (3) du dispositif est réalisée en un matériau transparent à au moins 30% de transmission du rayonnement UV.

3. Dispositif médical tel que notamment une sonde ETO selon la revendication 1 ou 2, **caractérisé en ce que** ladite partie (3) du dispositif permet de réduire d'au moins 15% les zones d'ombre du dispositif.

4. Dispositif médical tel que notamment une sonde ETO selon la revendication 1, 2 ou 3, **caractérisé en ce que** ladite partie (3) du dispositif est réalisée en quartz.

5. Dispositif médical tel que notamment une sonde ETO selon la revendication 1, 2 ou 3, **caractérisé en ce que** ladite partie (3) du dispositif est réalisée en polyfluoroéthylène.

6. Dispositif médical tel que notamment une sonde ETO selon l'une quelconque des revendications précédentes, **caractérisé en ce que** ladite partie (3) du dispositif comprend au moins un organe de manipulation de ce dispositif.

7. Dispositif médical tel que notamment une sonde ETO selon l'une quelconque des revendications précédentes, **caractérisé en ce qu'**il est adapté pour atteindre la désinfection DNI/HLD en moins de 10 mn dans un système à chambre UV qui délivre une dose cumulée d'au moins 60 mJ/cm² sur au moins une des autres surfaces du dispositif durant le cycle de désinfection.

8. Dispositif médical tel que notamment une sonde ETO selon l'une quelconque des revendications précédentes, **caractérisé en ce que** ladite partie du dispositif permet de réduire les écarts d'exposition de ses différentes portions de plusieurs ordres de grandeur.

9. Procédé de conception d'un dispositif médical tel que notamment une sonde ETO selon l'une quelconque des revendications précédentes, **caractérisé en ce qu'**il consiste à caractériser les zones d'ombre du dispositif, et à concevoir et valider la conception de celui-ci à l'aide d'un outil de simulation optique assistée par ordinateur.
